Europäisches Patentamt

European Patent Office

Office européen des brevets

(19)

(11) Veröffentlichungsnummer : **0 017 118
B1**

(12) **EUROPÄISCHE PATENTSCHRIFT**

(45) Veröffentlichungstag der Patentschrift :
03.02.82

(51) Int. Cl.³ : **C 07 C   7/08, C 07 C 11/12**

(21) Anmeldenummer : 80101531.4

(22) Anmeldetag : 22.03.80

(54) **Verfahren zur Gewinnung eines konjugierten Diolefins aus einem C 4- oder C 5-Kohlenwasserstoffgemisch.**

(30) Priorität : 23.03.79 DE 2911395

(43) Veröffentlichungstag der Anmeldung :
15.10.80 (Patentblatt 80/21)

(45) Bekanntmachung des Hinweises auf die Patenterteilung : 03.02.82 Patentblatt 82/05

(84) Benannte Vertragsstaaten :
AT BE CH DE FR GB IT NL SE

(56) Entgegenhaltungen :
DE - A - 2 165 453
DE - A1 - 2 738 418
GB - A - 1 340 149
US - A - 3 317 627

(73) Patentinhaber : BASF Aktiengesellschaft
Carl-Bosch-Strasse 38
D-6700 Ludwigshafen (DE)

(72) Erfinder : Volkamer, Klaus, Dr.
Heidelberger Ring 21
D-6710 Frankenthal (DE)
Erfinder : Broellos, Klaus, Dr.
Floriansring 7
D-6101 Seeheim (DE)
Erfinder : Lindner, Alfred, Dr.
Ringstrasse 22
D-6712 Bobenheim-Roxheim 1 (DE)
Erfinder : Wagner, Ulrich, Dr.
Knospstrasse 7
D-6703 Limburgerhof (DE)
Erfinder : Weitz, Hans-Martin, Dr.
Auf dem Koeppel 40
D-6702 Bad Duerkheim 1 (DE)
Erfinder : Schneider, Klaus, Juergen, Dr.
Triftbrunnenweg 16
D-6730 Neustadt 19 (DE)

0 017 118

# Verfahren zur Gewinnung eines konjugierten Diolefins aus einem $C_4$- oder $C_5$-Kohlenwasserstoffgemisch

Die vorliegende Erfindung betrifft ein Verfahren zur Gewinnung eines konjugierten Diolefins aus einem $C_4$- oder $C_5$-Kohlenwasserstoffgemisch durch extraktive Destillation mit Hilfe eines selektiven Lösungsmittels.

Die extraktive Destillation ist ein bekanntes Verfahren zur Trennung von Gemischen, die durch übliche fraktionierte Destillation nicht leicht trennbar sind, z.B. wenn die zu trennenden Komponenten ein Azeotrop bilden oder geringe Unterschiede in den relativen Flüchtigkeiten besitzen. Bei der extraktiven Destillation wird in die Destillationskolonne eine solche Menge eines relativ schwer flüchtigen Lösungsmittels eingeführt, daß die Unterschiede in den relativen Flüchtigkeiten der zu trennenden Komponenten erhöht werden und damit eine destillative Trennung ermöglicht wird. Typische Anwendungsbeispiele für die extraktive Destillation finden sich beispielsweise in C.S. Robinson et al « Elements of Fractional Distillation » 4. Auflage McGraw-Hill Book Company, Inc., New York, (1959), Seite 291.

Es ist bekannt, z.B. aus der DE-OS 27 42 148, DE-AS 15 68 902, DE-PS 11 63 795 oder aus The Soviet Chemical Industry, Nr. 11, November 1971, Seiten 719 bis 723, konjugierte Diolefine aus einem $C_4$- oder $C_5$-Kohlenwasserstoffgemisch durch extraktive Destillation unter Verwendung eines selektiven Lösungsmittels zu gewinnen. Die selektiven Lösungsmittel können wasserfrei verwendet werden. Bei dieser Arbeitsweise, die besonders bei hydrolyseempfindlichen Lösungsmitteln angewendet wird, ist jedoch im allgemeinen die $C_4$- oder $C_5$-Kohlenwasserstoff-Selektivität nicht ausreichend. Den selektiven Lösungsmitteln ist daher zur Erhöhung der Selektivität und zur Erniedrigung des Siedepunkts Wasser zugemischt worden. Die Wasserzugabe zum selektiven Lösungsmittel hat jedoch den Nachteil, daß die Löslichkeit der $C_4$- oder $C_5$-Kohlenwasserstoffe im selektiven Lösungsmittel erniedrigt wird, so daß die Menge an umlaufendem selektivem Lösungsmittel in der Extraktionsanlage entsprechend erhöht ist. Außerdem kann durch die Wasserzugabe die Viskosität und damit der Bodenwirkungsgrad des selektiven Lösungsmittels ungünstig beeinflußt werden.

Aus der DE-OS 27 38 418 ist weiter ein Verfahren zur Trennung von $C_5$-Kohlenwasserstoffgemischen durch extraktive Destillation bekannt, bei dem zur Steigerung der Selektivität ein selektives Lösungsmittel aus 67 bis 98 Gew. % Acetonitril, dem als Zusatzstoff 2 bis 33 Gew. % Dimethylformamid, Dimethylsulfoxid, Morpholin, Furfurol, N-Methylpyrrolidon, 3-Methoxypropionitril, $\gamma$-Butyrolacton, Äthylenglykolmonomethyläther, Diäthylenglykolmonomethyläther, Äthylenglykolmonoäthyläther oder Diäthylenglykolmonoäthyläther zugesetzt worden sind, verwendet wird. Trotz der Erhöhung der Selektivität weist auch die Mischung aus Acetonitril und dem Zusatzstoff keine für jede Trennaufgabe ausreichende Selektivität auf und ist auch in anderen Lösungsmitteleigenschaften, z.B. im Lösevermögen für bei der extraktiven Destillation gebildete Polymerablagerungen, nicht zufriedenstellend.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung eines Konjugierten Diolefins aus einem dieses enthaltenden $C_4$- oder $C_5$-Kohlenwasserstoffgemisch durch ein- oder mehrstufige extraktive Destillation mit einem selektiven Lösungsmittel, welches dadurch gekennzeichnet ist, daß man als selektives Lösungsmittel eine Lösungsmittelmischung verwendet, die

a) 75 bis 98 Gewichtsprozent eines N-alkylsubstituierten niederen aliphatischen Säureamids oder eines N-alkyl-substituierten alicyclischen Säureamids mit 5 Ringgliedern mit einem Siedepunkt im Bereich von 140 °C bis 260 °C oder Butyrolacton, Furfurol oder Methoxypropionitril und

b) 2 bis 25 Gewichtsprozent Acetonitril enthält.

Die Löslichkeit der $C_4$- oder $C_5$-Kohlenwasserstoffe in der erfindungsgemäß als selektives Lösungsmittel zu verwenden Lösungsmittelmischung ist gegenüber den bekannten Verfahren bei vergleichbar guter $C_4$- oder $C_5$-Kohlenwasserstoff-Selektivität wesentlich erhöht, so daß die Menge an umlaufendem selektivem Lösungsmittel in der Extraktionsanlage zur Gewinnung des konjugierten Diolefins stark erniedrigt werden kann. Dies hat insbesondere eine starke Senkung der Investitionskosten für die Extraktionsanlage und der Verbräuche an Dampf und elektrischer Energie zur Folge. Außerdem weist die erfindungsgemäß zu verwendende Lösungsmittelmischung eine geringere Viskosität, eine geringere Verdampfungswärme und eine geringere spezifische Wärme als die bekannten selektiven Lösungsmittel mit vergleichbar guter $C_4$- oder $C_5$-Kohlenwasserstoff-Selektivität . auf. Die geringere Viskosität der erfindungsgemäß zu verwendenden Lösungsmittelmischung bewirkt einen besseren Bodenwirkungsgrad in den extraktiven Destillationskolonnen, während durch die geringere Verdampfungswärme und geringere spezifische Wärme zusätzlich Energie eingespart werden kann.

Ein weiterer Vorteil der Verwendung einer Mischung eines der relativ hoch siedenden Lösungsmittel gemäß der vorstehenden Ziffer a) mit dem gemäß vorstehender Ziffer b) zu verwendenden, relativ niedrig siedenden Acetonitril besteht darin, daß die beispielsweise als Ausgaser oder als Lösungsmittelabstreifer betriebene Lösungsmittelwiedergewinnungszone der Extraktionsanlage zur Gewinnung des konjugierten Diolefins bei jeweisl gleicher Sumpftemperatur bei höherem Druck als mit den reinen unter Ziffer a) genannten Lösungsmitteln nach den bekannten Verfahren betrieben werden kann. Dies hat beispielsweise den Vorteil, daß die in der Lösungsmittelgewinnungszone erhaltenen ausgegasten Kohlenwasser-

2

stoffe aufgrund des höheren Drucks in einfacher Weise ohne Zwischenschaltung eines Kompressors oder Gebläses in nachgeschaltete, unter erhöhtem Druck betriebene Zonen gefördert werden können. Ein anderer Vorteil der Verwendung einer Mischung eines der relativ hoch siedenden Lösungsmittel gemäß der vorstehenden Ziffer a) mit dem gemäß vorstehender Ziffer b) zu verwendenden, relativ niedrig siedenden Acetonitril besteht darin, daß die z.B. als Ausgaser oder als Lösungsmittelabstreifer betriebene Lösungsmittelwiedergewinnungszone der Extraktionsanlage bei Anwendung des gleichen Drucks wie nach den bekannten Verfahren bei einer niedrigeren Sumpftemperatur betrieben werden kann, so daß eine Verschmutzung der Extraktionsanlage durch Polymerbildung leichter vermieden werden kann.

Es war überraschend, daß die erfindungsgemäß zu verwendende Lösungsmittelmischung eine erhöhte Selektivität besitzt und beispielsweise die Mischung aus N-Methylpyrrolidon und dem Zusatzstoff Acetonitril eine ebenso gute Kohlenwasserstoff-Selektivität aufweist wie die bisher verwendete Mischung aus N-Methylpyrrolidon und Wasser, da bei der Verwendung von Acetonitril als selektives Lösungsmittel für die Gewinnung von konjugierten Diolefinen dieses erst nach Zugabe von Wasser (vgl. z.B. US-PS 33 17 627) oder geringen Mengen von organischen Lösungsmitteln (vgl. DE-OS 27 38 418) eine ausreichende Kohlenwasserstoff-Selektivität erhält.

Für das Verfahren nach der Erfindung wird eine Lösungsmittelmischung verwendet, die

a) 75 bis 98 Gewichtsprozent eines N-alkylsubstituierten niederen aliphatischen Säureamids oder eines N-alkylsubstituierten alicyclischen Säureamids mit 5 Ringgliedern mit einem Siedepunkt im Bereich von 140 °C bis 260 °C oder Butyrolacton, Furfurol oder Methoxypropionitril und

b) 2 bis 25 Gewichtsprozent Acetonitril enthält.

Geeignete selektive Lösungsmittel gemäß vorstehender Ziffer a) sind Butyrolacton, Furfurol, Methoxypropionitril und vorzugsweise N-alkylsubstituierte niedere aliphatische Säureamide wie Dimethylformamid, Diäthylformamid, Dimethylacetamid, Diäthylacetamid oder Formylmorpholin und N-alkylsubstituierte cyclische aliphatische Säureamide (Lactame) mit 5 Riggliedern wie N-alkylpyrrolidone mit 1 bis 3 Kohlenstoffatomen im Alkylrest, insbesondere N-Methylpyrrolidon. Mit besonderem Vorteil wird von den Lösungsmitteln gemäß vorstehender Ziffer a) Dimethylformamid und insbesondere N-Methylpyrrolidon verwendet.

Die erfindungsgemäß zu verwendende Lösungsmittelmischung enthält 2 bis 25 Gewichtsprozenz, vorzugsweise 3 bis 20 Gewichtsprozent, an einem der organischen Lösungsmittel gemäß vorstehender Ziffer b). Entsprechend weist die Lösungsmittelmischung im allgemeinen einen Gehalt von 75 bis 98 Gewichtsprozent, vorzugsweise 80 bis 97 Gewichtsprozent, an Acetonitril auf. Mit besonderem Vorteil wird eine Lösungsmittelmischung gemäß der Erfindung verwendet, die 6 bis 15 Gewichtsprozent Acetonitril enthält.

Die erfindungsgemäß zu verwendende Lösungsmittelmischung kann einen geringen Wassergehalt, z.B. bis zu 10 Gewichtsprozent, aufweisen. Zweckmäßig wird jedoch der Wassergehalt auf höchstens 5 Gewichtsprozent, vorzugsweise höchstens 3 Gewichtsprozent, bezogen auf die Lösungsmittelmischung begrenzt. Es kann jedoch auch vorteilhaft sein, im wesentlichen wasserfrei, d.h. mit einem Wassergehalt von höchstens 1 Gewichtsprozent, vorzugsweise höchstens 0,5 Gewichtsprozent, insbesondere höchstens 0,1 Gewichtsprozent, bezogen auf die Lösungsmittelmischung, zu arbeiten.

Da Acetonitril mit Wasser ein azeotropes Gemisch bildet, kann es weiter vorteilhaft sein, eine erfindungsgemäße Lösungsmittelmischung mit einem Wassergehalt anzuwenden, der etwa dem azeotropen Verhältnis von Wasser zu Acetonitril, bezogen auf das in der Lösungsmittelmischung enthaltene Acetonitril, entspricht. Bei Wasserzugabe zur erfindungsgemäßen Lösungsmittelmischung beträgt das Gewichtsverhältnis von Wasser zu Acetonitril in der Lösungsmittelmischung zweckmäßig 1 : 3 bis 1 : 9, vorzugsweise 1 : 4 bis 1 : 8.

Die Gewinnung eines konjugierten Diolefins aus einem dieses enthaltenden $C_4$- oder $C_5$-Kohlenwasserstoffgemisch unter Verwendung der erfindungsgemäß als selektives Lösungsmittel einzusetzenden Lösungsmittelmischung erfolgt in an sich bekannter Weise (vgl. z.B. DE-PS 11 84 334, DE-AS 15 68 876, DE-AS 15 68 902) durch ein- oder mehrstufige, zweckmäßig ein- oder zweistufige extraktive Destillation. Die Gewinnung der konjugierten Diolefine, wie 1,3-Butadien, Isopren und 1,3-Pentadien aus der $C_4$- oder $C_5$-Kohlenwasserstoffmischung erfolgt beispielsweise in der Weise, daß das $C_4$- oder $C_5$-Kohlenwasserstoffgemisch, welches löslichere Kohlenwasserstoffe als das konjugierte Diolefin und weniger lösliche Kohlenwasserstoffe als das konjugierte Diolefin enthält, einer extraktiven Destillation mit dem erfindungsgemäß zu verwendenden Lösungsmittelgemisch unterworfen wird, wobei ein die weniger löslichen Kohlenwasserstoffe enthaltendes Destillat und ein das konjugierte Diolefin und die löslicheren Kohlenwasserstoffe und das selektive Lösungsmittel enthaltender Extrakt abgetrennt werden. Aus dem Extrakt kann das konjugierte Diolefin als Rohprodukt isoliert werden, das für manche Verwendungszwecke eine ausreichende Reinheit aufweist, das jedoch auch noch weiteren Reinigungsoperationen, z.B. einer fraktionierten Destillation, unterworfen werden kann. Vorteilhaft wird das konjugierte Diolefin jedoch unter Anwendung von zwei hintereinandergeschalteten extraktiven Destillationsstufen unter Verwendung der erfindungsgemäß zu verwendenden Lösungsmittelmischung gewonnen.

Hierbei werden beispielsweise — wie bereits vorstehend beschrieben — in der ersten Stufe der extraktiven Destillation ein die weniger löslichen Kohlenwasserstoffe enthaltendes Destillat und ein das konjugierte Diolefin und die löslicheren Kohlenwasserstoffe und das selektive Lösungsmittel enthaltender Extrakt abgetrennt. Dieser Extrakt wird vom selektiven Lösungsmittel befreit, wobei ein Gemisch aus dem konjugierten Diolefin und den löslicheren Kohlenwasserstoffen erhalten wird. Dieses Gemisch wird einer zweiten extraktiven Destillation mit dem selektiven Lösungsmittel unterworfen, wobei das konjugierte Diolefin als Destillat und ein Extrakt erhalten werden, der die löslicheren Kohlenwasserstoffe und das selektive Lösungsmittel enthält. Der erhaltene Extrakt wird anschließend vom selektiven Lösungsmittel befreit, wobei ein die löslicheren Kohlenwasserstoffe enthaltender Kohlenwasserstoffstrom erhalten wird.

Als konjugierte Diolefine enthaltendes Kohlenwasserstoffgemisch, das als Ausgangsgemisch für das Verfahren der vorliegenden Erfindung geeignet ist, kann eine $C_4$- oder $C_5$-Fraktion, die durch thermisches Kracken einer Petroleumfraktion (z.B. LPG, Naphtha usw.) erhalten wurde, eine durch Dehydrierung von n-Butan und/oder n-Buten erhaltene Butadien enthaltende Fraktion und eine durch Dehydrierung von Isopentan und/oder Isoamylen erhaltene Isopren enthaltende Fraktion verwendet werden. Im allgemeinen enthält das $C_4$-Kohlenwasserstoffgemisch 1,3-Butadien als konjugiertes Diolefin, Butane, n-Buten, Isobuten, Vinylacetylen, Äthylacetylen, 1,2-Butadien und gegebenenfalls geringe Mengen $C_3$- und/oder $C_5$-Kohlenwasserstoffe. Das $C_5$-Kohlenwasserstoffgemisch enthält in der Regel Isopren, trans- und cis-1,3-Pentadien und Cyclopentadien als konjugierte Diolefine sowie Pentane, n-Pentene, Isoamylen, Cyclopenten, höhere Acetylene.

Beispielsweise ergibt die extraktive Destillation einer $C_4$-Fraktion zunächst ein die Butane und Butene enthaltendes Destillat sowie einen 1,3-Butadien, Äthylacetylen, Vinylacetylen und 1,2-Butadien enthaltenden Extrakt, der, wenn er einer weiteren extraktiven Destillation unterworfen wird, als Destillat 1,3-Butadien liefert, während der Extrakt Äthylacetylen, Vinylacetylen und 1,2-Butadien enthält. Aus dem Äthylacetylen, Vinylacetylen und 1,2-Butadien enthaltenden Extrakt werden diese Kohlenwasserstoffe in einem Ausgaser abgetrennt und das entgaste Lösungsmittel in die extraktive Destillation zurückgeführt. Das als Destillat erhaltene 1,3-Butadien kann anschließend zur Abtrennung von gegebenenfalls noch vorhandenen sehr geringen Mengen von $C_3$- und/oder $C_5$-Kohlenwasserstoffen einer fraktionierten Destillation unterworfen werden.

Die nachstehenden Beispiele dienen der weiteren Erläuterung der Erfindung :

### Beispiel 1

In einer Butadien-Gewinnungsanlage mit zwei hintereinandergeschalteten Extraktivdestillationszonen wird ein $C_4$-Kohlenwasserstoffgemisch ($C_4$-Fraktion aus einer Äthylenanlage) der in Tabelle 1 aufgeführten Zusammensetzung aufgetrennt, wobei in der ersten Extraktivdestillationszone ein die Butane und Butene enthaltendes Destillat sowie ein Butadien-1,3, Äthylacetylen, Vinylacetylen und Butadien-1,2 enthaltender Extrakt erhalten werden. Der Extrakt wird einer zweiten extraktiven Destillation unterworfen, die als Destillat Butadien-1,3 liefert, während der Extrakt Äthylacetylen, Vinylacetylen und Butadien-1,2 enthält. Der Extrakt wird zur Ausgasung der Kohlenwasserstoffe einem Ausgaser zugeführt, in dem als Sumpfprodukt das entgaste selektive Lösungsmittel und als Kopfprodukt ein Vinylacetylen, Äthylacetylen und Butadien-1,2 enthaltender Kohlenwasserstoffstrom erhalten wird, der als Abfallstrom z.B. einem Kraftwerk zur Verbrennung zugeführt wird.

Tabelle 1

| Zusammensetzung | Gew.% |
|---|---|
| Propan | 0,03 |
| Propen | 0,10 |
| Propadien | 0,02 |
| Propin | 0,15 |
| n-Butan | 3,1 |
| i-Butan | 1,0 |
| Buten-1 | 14,0 |
| i-Buten | 27,0 |
| Buten-2-trans | 5,0 |
| Buten-2-cis | 4,3 |
| Butadien-1,3 | 44,0 |
| Butadien-1,2 | 0,2 |
| Äthylacetylen | 0,2 |
| Vinylacetylen | 0,7 |
| $C_5$-Kohlenwasserstoffe | 0,2 |

Als selektives Lösungsmittel wird eine Mischung aus 88 Gew. % N-Methylpyrrolidon (NMP) 10 Gew. % Acetonitril (ACN) und 2 Gew. % Wasser (Lösungsmittel nach der Erfindung) verwendet, in der die Gehalte an ACN und Wasser dem azeotropen ACN/Wasser-Gemisch bei 1 bar entsprechen.

In einem Vergleichsversuch wird anstelle des Lösungsmittels nach der Erfindung eine dem Stand der Technik entsprechende Mischung aus 91,7 Gew. % NMP und 8,3 Gew. % Wasser (Vergleichslösungsmittel) verwendet.

Für das Lösungsmittel nach der Erfindung und das Vergleichslösungsmittel ergeben sich die in Tabelle 2 aufgeführten Kohlenwasserstoffselektivitäten (bezogen auf Butadien-1,3 gleich 1) :

Tabelle 2

|  | i-Buten | Buten-2 | | Buta-dien-1,3 | Äthyl-acetylen |
| --- | --- | --- | --- | --- | --- |
|  |  | trans | cis |  |  |
| 91,7 Gew.% NMP/ 8,3 Gew.% $H_2O$ | 2,63 | 2,17 | 1,85 | 1 | 0,36 |
| 88 Gew.% NMP/ 10 Gew.% ACN/ 2 Gew.% $H_2O$ | 2,58 | 2,20 | 1,82 | 1 | 0,36 |

Die Selektivitäten für die Schlüsselkomponente der ersten extraktiven Destillation (Buten-2-cis) und die Schlüsselkomponente der zweiten extraktiven Destillation (Äthylacetylen) sind für das Vergleichslösungsmittel und das Lösungsmittel nach der Erfindung innerhalt der Meßgenauigkeit gleich. (Die Selektivität ist das Verhältnis der Bunsenschen Absorptionskoeffizienten von Butadien-1,3 und abzutrennendem Kohlenwasserstoff.).

Das Lösungsmittel nach der Erfindung weist bei 4,5 bar für das $C_4$-Kohlenwasserstoffgemisch gegenüber dem Vergleichslösungsmittel eine um ca. 60 % höhere Löslichkeit auf. Daher kann die zur ersten und zweiten extraktiven Destillation zuzuführende Menge an selektivem Lösungsmittel bei Verwendung des Lösungsmittels nach der Erfindung anstelle des Vergleichslösungsmittels entsprechend um ca. 60 % reduziert werden. Hierdurch verringern sich die Kolonnendurchmesser der extraktiven Destillationskolonnen um etwa 30 %. Da die Bauhöhen der extraktiven Destillationskolonnen außerdem aufgrund des nachstehend beschriebenen erhöhten Bodenwirkungsgrades des Lösungsmittels nach der Erfindung um ca. 25 % erniedrigt werden können, können die Aufwendungen bezüglich der Apparatekosten für die extraktiven Destillationskolonnen bei Verwendung des Lösungsmittels nach der Erfindung um mehr als 50 % gesenkt werden.

Gleichzeitig kann durch die bei Verwendung des Lösungsmittels nach der Erfindung reduzierte Menge an dem den extraktiven Destillationen zuzuführenden selektiven Lösungsmittel der Dampfverbrauch um ca. 10 % reduziert werden.

Das Lösungsmittel nach der Erfindung weist gegenüber dem Vergleichslösungsmittel einen um 27 % erhöhten Wirkungsgrad für die Böden der Extraktivdestillationskolonnen auf. Hierdurch können bei der Verwendung des Lösungsmittels nach der Erfindung im Vergleich zum Vergleichslösungsmittel zur Erzielung des gleichen Trennergebnisses Extraktivdestillationskolonnen mit um ca. 25 % erniedrigter Bauhöhe verwendet werden.

In dem für die Ausgasung wesentlichen Temperaturbereich zwischen 130 und 160 °C sind die Dampfdrücke des Lösungsmittels nach der Erfindung und des Vergleichslösungsmittels praktisch gleich. Bei beispielsweise 150 °C wird bei der Ausgasung des nach der zweiten extraktiven Destillation erhaltenen Extraktes im Ausgaser ein Druck von ca. 1,7 bar erreicht, der hinreichend hoch ist, um das die $C_4$-Acetylene (Vinylacetylen, Äthylacetylen ) enthaltende Abgasgemisch unmittelbar ohne Zwischenschaltung eines Kompressors oder Gebläses einem Kraftwerke oder einer unter üblichem Druck betriebenen Abgasfackel zuführen zu können. Eine spezielle Niederdruckfackel ist daher bei der Verbrennung in einer Abgasfackel nicht erforderlich.

Beispiel 2

Dieses Beispiel veranschaulicht die einstufige extraktive Destillation eines $C_4$-Kohlenwasserstoffgemisches.

Einer bei 1 bar und 15 °C betriebenen Füllkörperkolonne mit 25 mm Innendurchmesser und 2,50 m Höhe werden am Sumpf 0,716 kg/h $C_4$-Kohlenwasserstoffgemisch der folgenden Zusammensetzung zugeführt :

| Zusammensetzung | Gew.% |
| --- | --- |
| i-Butan | 1,33 |
| n-Butan | 4,44 |
| Buten-1 | 11,65 |
| i-Buten | 28,21 |

(Fortsetzung)

| Zusammensetzung | Gew.% |
|---|---|
| Buten-2-trans | 7,28 |
| Buten-2-cis | 4,45 |
| Butadien-1,3 | 41,98 |
| Butadien-1,2 | 0,31 |
| Äthylacetylen | 0,24 |
| Vinylacetylen | 0,11 |

Am Kopf der Kolonne werden 4,62 kg/h im Kreise geführtes selektives Lösungsmittel von 15 °C aus 88 Gewichtsprozent N-Methylpyrrolidon, 10 Gewichtsprozent Acetonitril und 2 Gewichtsprozent Wasser zugegeben. 0,374 kg/h Raffinat mit einem Gehalt an Butadien-1,3 von 7,46 Gewichtsprozent und an Buten-2-cis (Schlüsselkomponente für die Trennung) von 5,61 Gewichtsprozent werden am Kopf der Kolonne gasförmig abgezogen. Butadien-1,2 und die $C_4$-Acetylene (Äthylacetylen, Vinylacetylen) sind im Raffinat nicht mehr nachweisbar. Am Sumpf der Kolonne wird ein die Leichter löslichen Kohlenwasserstoffe enthaltender Extrakt abgezogen, der zur Ausgasung dem Kopf einer nachgeschalteten Kolonne mit 10 Glockenböden zugeführt wird, deren Sumpftemperatur auf 130 bis 140 °C gehalten wird. Vom Sumpf der Glockenbodenkolonne wird das weitgehend von den Kohlenwasserstoffen befreite selektive Lösungsmittel nach Abkühlung auf den Kopf der Füllkörperkolonne zurückgeführt. In der Mitte der Glockenbodenkolonne werden 0,342 kg/h Rohbutadien mit einem Butadien-1,3-Gehalt von 79,80 Gewichtsprozent abgezogen. Die am Kopf der Glockenbodenkolonne austretenden Kohlenwasserstoffe werden gasförmig in den Sumpf der Füllkörperkolonne zurückgeführt.

Vergleichsversuch

In einem Vergleichsversuch verfährt man wie im vorstehenden Beispiel 2 beschrieben, wobei jedoch als selektives Lösungsmittel eine Mischung aus 91,7 Gewichtsprozent N-Methylpyrrolidon und 8,3 Gewichtsprozent Wasser verwendet wird, die der Füllkörperkolonne in einer gegenüber dem Beispiel 2 erhöhten Menge von 6,00 kg/h zugeführt wird, und das $C_4$-Kohlenwasserstoffgemisch in einer gegenüber dem Beispiel 2 erniedrigten Menge von 0,471 kg/h am Sumpf der Füllkörperkolonne zugegeben wird, wobei 0,235 kg/h Raffinat und 0,236 kg/h Rohbutadien erhalten werden. Obwohl im Vergleichsversuch das Verhältnis von zugeführtem selektiven Lösungsmittel zu zugeführtem $C_4$-Kohlenwasserstoffgemisch (12, 74) gegenüber dem entsprechenden Verhältnis im Beispiel 2 (6, 45) nahezu verdoppelt ist, wird im Vergleichsversuch bei praktisch gleichem Verhältnis der Menge an Raffinat und der Rohbutadienmenge (0,235/0,236) wie im Beispiel 2 (0,374/0,342) ein Raffinat mit einem Butadien-1,3-Gehalt von 14,77 Gewichtsprozent erhalten gegenüber einem Butadien-1,3-Gehalt im Raffinat gemäß Beispiel 2 von nur 7,46 Gewichtsprozent. Außerdem wird die Schlüsselkomponente Buten-2-cis im Raffinat gemäß dem Vergleichsversuch (4,41 Gew. % Buten-2-cis) weniger stark angereichert als im Raffinat gemäß Beispiel 2 (5,61 Gewichtsprozent Buten-2-cis). Während Butadien-1,2, Äthylacetylen und Vinylacetylen mit einem Gehalt im Raffinat gemäß dem Vergleichsversuch noch deutlich nachweisbar sind, liegen die Gehalte an diesen Komponenten im Raffinat gemäß Beispiel 2 unter der Nachweisbarkeitsgrenze. Außerdem weist das im Vergleichsversuch erhaltene Rohbutadien lediglich einen Butadien-1,3-Gehalt von 69,07 Gewichtsprozent auf gegenüber einem Butadien-1,3-Gehalt im Rohbutadien gemäß Beispiel 2 von 79,80 Gewichtsprozent.

**Ansprüche**

1. Verfahren zur Gewinnung eines konjugierten Diolefins aus einem dieses enthaltenden $C_4$- oder $C_5$-Kohlenwasserstoffgemisch durch ein- oder mehrstufige extraktive Destillation mit einem selektiven Lösungsmittel, dadurch gekennzeichnet, daß man als selektives Lösungsmittel eine Lösungsmittelmischung verwendet, die
a) 75 bis 98 Gewichtsprozent eines N-alkylsubstituierten niederen aliphatischen Säureamids oder eines N-alkylsubstituierten alicyclischen Säureamids mit 5 Ringgliedern mit einem Siedepunkt im Bereich von 140 °C bis 260 °C oder Butyrolacton, Furfurol oder Methoxypropionitril und
b) 2 bis 25 Gewichtsprozent Acetonitril enthält.
2. Verfahren nach Anspruch 1, dadurch gekennzeichnet, daß man eine Lösungsmittelmischung verwendet, die
a) 75 bis 98 Gewichtsprozent Dimethylformamid, Diäthylformamid, Dimethylacetamid, diäthylacetamid, Formylmorpholin, N-Methylpyrrolidon, Butyrolacton, Furfurol oder Methoxypropionitril und
b) 2 bis 25 Gewichtsprozent Acetonitril enthält.

3. Verfahren nach Anspruch 1 und 2, dadurch gekennzeichnet, daß die Lösungsmittelmischung höchstens 5 Gewichtsprozent Wasser, bezogen auf die Lösungsmittelmischung enthält.

**Claims**

1. A process for isolating a conjugated diolefin from a $C_4$- or $C_5$-hydrocarbon mixture containing the diolefin, by single-stage or multi-stage extractive distillation using a selective solvent, characterized in that there is used as selective solvent a solvent mixture which comprises

a) from 75 to 98 per cent by weight of an N-alkyl-substituted lower aliphatic acid amide or of an N-alkyl-substituted alicyclic acid amide having 5 ring members, boiling at from 140° to 260°C, or butyrolactone, furfuraldehyde or methoxypropionitrile, and

b) from 2 to 25 per cent by weight of acetonitrile.

2. A process as claimed in claim 1, characterized in that a solvent mixture is used which comprises

a) 75 to 98 per cent by weight of dimethylformamide, diethylformamide, dimethylacetamide, diethylacetamide, formylmorpholine, N-methylpyrrolidone, butyrolactone, furfuraldehyde or methoxypropionitrile, and

b) from 2 to 25 per cent by weight of acetonitrile.

3. A process as claimed in claims 1 and 2, characterized in that the solvent mixture contains at most 5 per cent by weight of water, based on the solvent mixture.

**Revendications**

1. Procédé pour l'obtention d'une dioléfine conjuguée à partir d'un mélange d'hydrocarbures à 4 ou 5 C contenant celle-ci, par une distillation extractive en un ou en plusieurs temps avec un solvant sélectif, caractérisé en ce qu'on utilise, en tant que solvant sélectif, un mélange de solvants qui contient :

a) 75 à 98 % en poids d'un amide d'acide aliphatique inférieur N-substitué par un alcoyle ou d'un amide d'acide alicyclique N-substitué par un alcoyle à 5 termes cycliques, ayant un point d'ébullition dans la gamme de 140 °C à 260 °C, ou de butyrolactone, de furfural ou de méthoxypropionitrile, et

b) 2 à 25 % en poids d'acétonitrile.

2. Procédé selon la revendication 1, caractérisé en ce qu'on utilise un mélange de solvants qui contient :

a) 75 à 98 % en poids de diméthylformamide, de diéthylformamide, de diméthylacétamide, de diéthylacétamide, de formylmorpholine, de N-méthylpyrrolidone, de butyrolactone, de furfural ou de méthoxypropionitrile, et

b) 2 à 25 % en poids d'acétonitrile.

3. Procédé selon la revendication 1 ou 2, caractérisé en ce que le mélange de solvants contient au maximum 5 % en poids d'eau, par rapport au mélange de solvants.